# EUROPÄISCHE PATENTANMELDUNG

(11) **EP 3 220 126 A1**
(43) Veröffentlichungstag der Anmeldung: **20.09.2017**
(21) Anmeldenummer: 16160850.0
(22) Anmeldetag: 17.03.2016
(51) Int. Cl.: G01N 11/08, G01N 33/44, B29C 47/92

(54) **VORRICHTUNG ZUR MESSUNG DER VISKOSITÄT VON FLÜSSIGKEITEN**

(71) Anmelder: iVON GmbH, 4073 Wilhering (AT)
(72) Erfinder: Fellinger, Markus, 4073 Wilhering (AT); Fellinger, Ingrid, 4073 Wilhering (AT)
(74) Vertreter: Margotti, Herwig Franz

(57) **Zusammenfassung**

Eine Vorrichtung zur Messung der Viskosität von Flüssigkeiten umfasst ein Gehäuse (1), in dem eine einlaufseitige Zahnradpumpe (3A) zur Förderung der Flüssigkeit angeordnet ist. Ein Antriebsmotor (20) treibt eine Welle (4), die ein Zahnrad der Zahnradpumpe (3A) antreibt. Stromabwärts von der einlaufseitigen Zahnradpumpe (3A) ist eine Messdüse (5) angeordnet, der ein einlaufseitiger Massedrucksensor (6A) vorgeschaltet ist. Das Gehäuse (1) besitzt einen Oberteil (1A), einen Mittelteil (1B) und einen Unterteil (1C), die als separate, lösbar miteinander verbindbare Gehäuseteile ausgebildet sind. Die einlaufseitige Zahnradpumpe (3A) ist entweder im Oberteil (1A) oder im Mittelteil (1B) angeordnet und die Messdüse (5) ist im Mittelteil (1B) angeordnet. Der Oberteil (1A), der Mittelteil (1B) und der Unterteil (1C) des Gehäuses (1) sind so konfiguuert, dass im getrennten Zustand die einlaufseitige Zahnradpumpe (3A) und die Messdüse (5) von außen zugänglich sind.

## Beschreibung

Die Erfindung bezieht sich auf die Messung der Viskosität von Flüssigkeiten, im speziellen der Messung von zähen Flüssigkeiten. Insbesondere bezieht sich die Erfindung auf die Viskositätsbestimmung von Kunststoffen, wie z.B. thermoplastischen Kunststoffschmelzen, beispielsweise Polyethylen, Polypropylen, Polystyrene, Polycarbonate, Polyester oder PET und Polyamide.

Die Viskositäten von Flüssigkeiten sind ein wesentliches Eigenschafts- und Qualitätsmerkmal der Flüssigkeiten und sind ausschlaggebend für die Einsatzbereiche, die Möglichkeiten über die weitere Verwendung und geben Aufschlüsse über prozesstechnische Verarbeitung, Mischungen etc. der Flüssigkeiten.

Auf dem Gebiet der Kunststoffverarbeitung, insbesondere bei der Verarbeitung von thermoplastischen Kunststoffen, geben die Viskositäten Auskunft über die prozesstechnischen und mechanischen Eigenschaften der Halbfertig- und/oder Endprodukte aus den Kunststoffen.

Der vorliegenden Erfindung liegt die Aufgabe zugrunde, die Viskosität von Flüssigkeiten, im Speziellen von Kunststoffschmelzen direkt während der Verarbeitung der Flüssigkeiten automatisch zu bestimmen.

In der Verarbeitung von Kunststoffschmelzen ist es üblich, den Schmelze/Gewichtsindex bzw. den Schmelze-Volumen-Index in einem genormten Verfahren nach z.B. ISO 1133-1 zu bestimmen. Dabei werden Kunststoffteile in der Form von Granulat, Mahlgut oder Pulver in einen beheizten Messapparat gefüllt, über eine vordefinierte Zeit aufgeschmolzen und dann mittels eines definierten konstanten Druckes durch eine Messdüse gepresst. Das Gewicht des pro Zeiteinheit durch die Messdüse gepressten Kunststoffes oder das Volumen des pro Zeiteinheit durch die Messdüse gepressten Kunststoffes gibt somit Auskunft über die Viskosität des gemessenen Materials. Dieses Verfahren kann auch für Kunststoffschmelzen, wie z.B. PET oder Polyamide, angewendet werden.

Ein weiteres Verfahren zur Viskositätsbestimmung von Kunststoffen, wie z.B. bei PET häufig angewandt, ist die Bestimmung der intrinsischen Viskosität. Dabei wird der Ausgangskunststoff mittels einer Säure verflüssigt und dann der Durchfluss der Kunststoff-Säure-Mischung durch eine Messkapillare pro Zeiteinheit bestimmt. Diese Messung ist z.B. aus DIN EN 15348 oder DIN EN ISO 1628-5 bekannt.

Diese oben beschrieben Bestimmungen der Zähigkeiten von Flüssigkeiten haben jedoch den Nachteil, dass zuerst Proben entnommen werden müssen und diese in der Regel von Laborpersonal zeitaufwendig bestimmt werden müssen. Ein Rückschluss auf und gegebenenfalls eine Änderung von Prozessparametern, Mischungsverhältnissen, Ausschuss etc. ist somit erst zeitverzögert möglich.

Es stellt sich daher die Aufgabe, die Viskosität von Flüssigkeiten direkt während der Verarbeitung möglichst zeitnah zu messen, um somit direkt in den Prozess eingreifen zu können (z.B. Prozessparameter, Rohstoffe oder Mischungsverhältnisse zu ändern).

Solche Vorrichtungen und Messeinrichtungen sind bekannt.

In US 4425790 wird ein Verfahren beschrieben, in dem eine Polymer-Schmelze durch mehrere aufeinanderfolgende Messdüsen mittels einer Zahnradpumpe bei konstantem Volumen Strom gedrückt wird und über den Druckabfall an den Messdüsen die "extrusion performance" vorausgesagt wird.

Das Dokument WO 97/10492 beschreibt ein Verfahren zur Auswertung von Schmelzeindexwerten mithilfe eines Online Kapillarrheometers.

Das Dokument WO 00/22410 beschreibt eine Vorrichtung zur Messung der Viskosität plastischer Massen.

Diese oben erwähnten Druckschriften haben gemeinsam, dass ein Schmelzestrom aus Kunststoffschmelze mit konstantem oder regelbarem Volumen durch eine oder mehrere Messdüsen geführt wird und über den Druckabfall bzw. bei konstantem Druck einem Volumenstrom rheologische Eigenschaften der Flüssigkeit zugeordnet werden.

All diese Verfahren oder Vorrichtungen offenbaren jedoch nicht die genaue Ausführung der Messmaschine beziehungsweise den praxisgerechten und störungsfreien Betrieb der Messmaschine.

Die erfindungsgemäße Viskositätsmessvorrichtung löst die gestellte Aufgabe durch Bereitstellen einer Vorrichtung mit den Merkmalen des Anspruchs 1. Vorteilhafte Ausgestaltungen der Erfindung sind in den Unteransprüchen und der nachfolgenden Beschreibung definiert.

Die Erfindung wird nun anhand von den Schutzbereich nicht einschränkenden Ausführungsbeispielen unter Bezugnahme auf die Zeichnungen im Detail beschrieben. In den Zeichnungen zeigen:
Fig. 1 eine schematische Darstellung der erfindungsgemäßen Viskositätsmessvorrichtung in Seitenansicht, angeschlossen an den Ausgang eines Extruders;
Fig. 2 einen Längsschnitt durch die erfindungsgemäße Viskositätsmessvorrichtung;
Fig. 3 ein Detail der erfindungsgemäßen Viskositätsmessvorrichtung im Längsschnitt;
Fig. 4 ein weiteres Detail der erfindungsgemäßen Viskositätsmessvorrichtung im Längsschnitt; und
Fig. 5 ein Detail der erfindungsgemäßen Viskositätsmessvorrichtung in Seitenansicht.

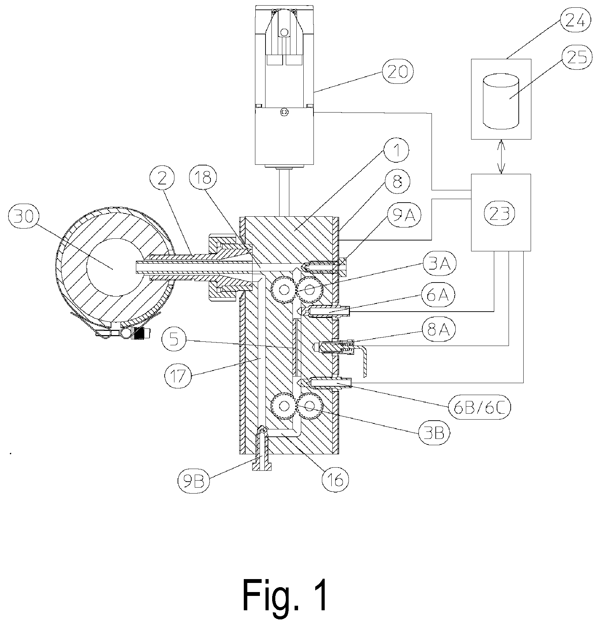

Es sei darauf hingewiesen, dass in der nachfolgenden Beschreibung von Ausführungsbeispielen der vorliegenden Erfindung unter Bezugnahme auf Fig. 1 bis Fig. 5 im Speziellen auf thermoplastische Kunststoffe eingegangen wird. Die Erfindung ist jedoch nicht auf thermoplastische Kunststoffe beschränkt, sondern ist auch für die Viskositätsmessung anderer zähflüssiger Flüssigkeiten geeignet.

Die Erfindung hat sich zur Aufgabe gesetzt, eine möglichst praxistaugliche Vorrichtung zur Viskositätsbestimmung von Flüssigkeiten bereitzustellen, die mit möglichst einfachen Mitteln, einfachen Herstellungsmethoden und mit Software ein reproduzierbares und verlässliches Messergebnis liefert.

Erfindungsgemäß ist die Viskositätsmessvorrichtung mit einem Zuführrohr versehen, durch das hindurch im Produktionsprozess eines Halbfertig- oder Fertigerzeugnisses eine Kunststoffschmelze an möglichst aussagekräftiger Stelle des Produktionsprozesses der Viskositätsmessvorrichtung zugeführt wird. Im Regelfall wird sich diese Stelle am Ende eines Extruders nach einer gegebenenfalls vorhandenen Filtriereinrichtung und nach einer oder mehreren optional vorhandenen Zahnradpumpen vor einer formgebenden Einrichtung befinden.

Als formgebende Einrichtungen kommen dabei beispielsweise Runddüsen zur Herstellung von Rohren oder Blasfolien, Werkzeuge zur kontinuierlichen Herstellung von Profilen wie z.B. Fensterprofilen, Granulierlochplatten, oder Düsen zur Herstellung von Granulaten, Breitflachdüsen zur Herstellung von Flachfolien oder Werkzeuge von Spritzgießmaschinen infrage. Durch den Rückdruck der formgebenden Einrichtung wird die Kunststoffschmelze in das, optional beheizte Zuführrohr in die Viskositätsmessvorrichtung zugeführt.

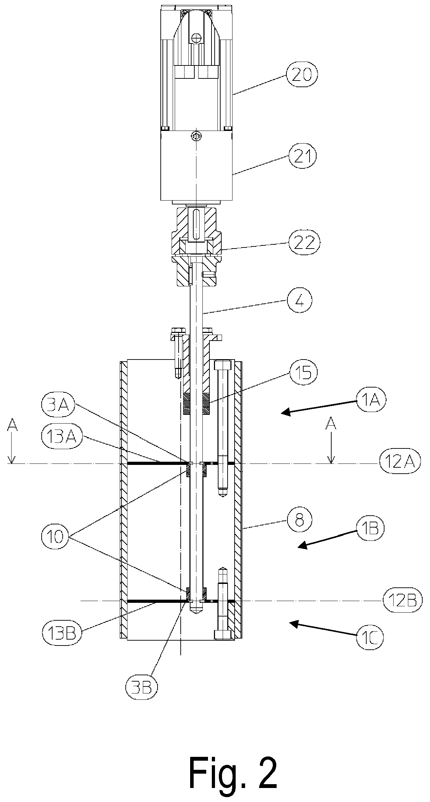

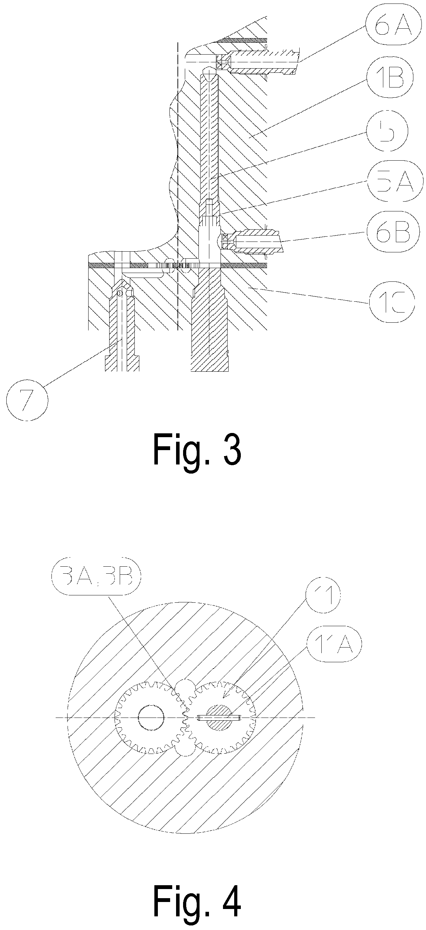

Die erfindungsgemäße Vorrichtung zur Viskositätsbestimmung von Flüssigkeiten weist ein Gehäuse 1 auf, das mindestens drei Teile umfasst, nämlich einen Oberteil 1A, in den das Zuführrohr 2 mündet, einen Mittelteil 1B und einen Unterteil 1C. Diese drei Teile sind als separate Gehäuseteile ausgebildet, wobei der Oberteil 1A mit dem Mittelteil 1B und der Mittelteil 1B mit dem Unterteil 1C lösbar verbindbar sind, z.B. durch Verschraubung. Zwei Zahnradpumpen 3A, 3B sind in das Gehäuse 1 der Viskositätsmessvorrichtung eingebaut, wobei eine der Zahnradpumpen 3A entweder in den Oberteil 1A oder den Mittelteil 1B und die andere Zahnradpumpe 3B entweder in den Mittelteil 1B oder den Unterteil 1C eingebaut ist. Es sei erwähnt, dass in der schematischen Darstellung von Fig. 1 zum besseren Verständnis der Strömungsverhältnisse die Zahnradpumpen 3A, 3B vertikal eingezeichnet sind. In der in Fig. 3 dargestellten praxiskonformen Ausführungsform sind die Zahnradpumpen 3A, 3B aufgrund konstruktiver Vereinfachungen jedoch horizontal angeordnet. Die drei Teile 1A, 1B, 1C des Gehäuses 1 sind so ausgebildet, dass im getrennten Zustand die Zahnradpumpen 3A, 3B frei liegen und für Ausbau, Einbau und Wartung von außen zugänglich sind. Im in Fig. 2 dargestellten Ausführungsbeispiel sind die drei Teile 1A, 1B, 1C in Ebenen 12A, 12B voneinander getrennt und an diesen Ebenen zum Gesamtgehäuse 1 zusammensetzbar und lösbar miteinander verbindbar. Die Gehäuseteile 1A, 1B, 1C können beispielsweise aus massiven Körpern aus Aluminium gefräst sein und Hohlräume zur Aufnahme der Zahnradpumpen 3A, 3B sowie weiterer nachfolgend beschriebener Elemente aufweisen. Auch die Leitungen für den Transport der zu messenden Kunststoffschmelze können in den Gehäuseteile mittels Fräsen und Bohren ausgebildet sein.

Jede der beiden Zahnradpumpen 3A, 3B weist ein Zahnradpaar mit einem angetriebenen Zahnrad und einem mitgetriebenen Zahnrad auf, wobei beide Zahnradpumpen von einer gemeinsamen, durch einen Motor 20 angetriebenen Welle 4 angetrieben werden. Der Eingang der einlaufseitigen Zahnradpumpe 3A kommuniziert mit dem Zuführrohr 2. Zwischen dem Ausgang der einlaufseitigen Zahnradpumpe 3A und dem Eingang der austragseitigen Zahnradpumpe 3B ist eine Messdüse 5 eingebaut, die auf dem technischen Gebiet auch unter dem Begriff "Messkapillare" bekannt ist. Die stromabwärts von der Messdüse 5 angeordnete austragseitige Zahnradpumpe 3B weist einen größeren Volumen pro Zeit Durchsatz auf als die stromaufwärts von der Messdüse 5 sitzende Zahnradpumpe 3A. Der Ausgang der austragseitigen Zahnradpumpe 3B kommuniziert mit einer Austragsleitung 16 und daran anschließend einer Rückführleitung 17.

Über die Welle 4 wird das Drehmoment in das angetriebene Zahnrad einer jeden Zahnradpumpe 3A, 3B eingeleitet. Bei konstanter Drehzahl der Welle 4 erzeugt die einlaufseitige Zahnradpumpe 3A einen konstanten Volumenstrom des eingeleiteten Kunststoffs, der vor der Messdüse 5 einen Druckaufbau erzeugt, der mittels eines einlaufseitigen Massedrucksensors 6A erfasst wird.

Die zwischen den Zahnradpumpen 3A, 3B angeordnete Messdüse 5 kann bei getrennten Teilen 1A, 1B, 1C einfach von außen getauscht werden, indem sie steckbar oder schraubbar im Mitteilteil 1B sitzt. Eventuell ist vor dem Tausch eine der Zahnradpumpen 3A, 3B zu entfernen. Die Geometrie der Messdüse 5 in Länge und Querschnitt kann auf die zu messende Flüssigkeit in Abhängigkeit von ihrer Viskosität abgestimmt werden. Dabei ist zu beachten, dass der von der Messdüse 5 erzeugte Druck einen möglichst aussagekräftigen Wert im erfassbaren Druckmessbereich des einlaufseitigen Massedrucksensors 6A ergibt, der nicht zu hoch oder zu niedrig sein soll, einerseits um etwaige Ungenauigkeiten des Messbereichs des Massedrucksensors 6A zu minimieren, andererseits um die Scherung und eine damit verbundene Schädigung des Kunststoffes in der Viskositätsmessvorrichtung zu limitieren, wodurch das Ergebnis verfälscht würde.

In der Praxis hat sich gezeigt, dass eine Messdüsengeometrie mit einem Druckaufbau von 5-100 bar bei gegebenem Volumenstrom zu guten, reproduzierbaren Ergebnissen führt.

Da die Viskositäten von verschiedenen Kunststoffen stark variieren und auf einer Kunststoff-Verarbeitungsmaschine oft verschiedene Materialien, wie z.B. HDPE und Polyester, verarbeitet werden können, ist es besonders vorteilhaft, dass diese Messdüse 5 einfach und ohne großen Aufwand schnell gewechselt werden kann. In der dargestellten Ausführungsform ist die Messdüse 5 mittels eines Gewindes 5A (siehe Fig. 3) in den Mittelteil 1B des Gehäuses 1 geschraubt. Es sind jedoch auch andere Ausführungsformen, z.B. mit einer gelochten Verschlussschraube 7 denkbar, die die Messdüse 5 über Pressung in Position hält.

Vor der zweiten, austragseitigen Zahnradpumpe 3B sitzt optional ein zweiter, austragseitiger Massedrucksensor 6B, der den sich einstellenden Vordruck vor der austragseitigen Zahnradpumpe 3B misst. Da die austragseitige Zahnradpumpe 3B eine größere Förderrate als die einlaufseitige Zahnradpumpe 3A besitzt, liegt der Druck am austragseitigen Massedrucksensor 6B im Betrieb annähernd im Bereich zwischen 0 und 5 bar und kann daher üblicherweise vernachlässigt werden. Der austragseitige Massedrucksensor 6B dient somit vor allem einer Erhöhung der Messgenauigkeit, indem für die Bestimmung der Viskosität die Druckdifferenz zwischen den beiden Massedrucksensoren 6A und 6B verwendet wird.

Der Querschnitt des Zuführrohrs 2, das auch aus mehreren parallel geschalteten Teil-Zuführrohren mit Teil-Querschnitten bestehen kann, die zu einem Gesamtquerschnitt summiert werden, wird im Verhältnis zum Querschnitt der Messdüse 5 im Regelfall so festgelegt, dass die Verweilzeit der Kunststoffschmelze unter Druck und Temperatur einerseits zu keinem oder einem nur sehr geringen Abbau des Kunststoffes und andererseits zu einer möglichst genauen Angleichung der Kunststoffschmelzetemperatur an die Gehäusetemperatur führt. Am Gehäuse 1 der Viskositätsmessvorrichtung ist eine Heizvorrichtung 8 angebracht, mit der die Gehäusetemperatur auf einen eingestellten Wert gebracht und gehalten wird, wobei die Gehäusetemperatur von einem Temperatursensor 8A kontinuierlich erfasst wird. Durch diese Maßnahmen wird einer Veränderung der Viskosität durch erhöhte Temperaturen der Kunststoffschmelze, hervorgerufen durch geänderte Verfahrensbedingungen im Extruder 30, wie z.B. höhere Schneckendrehzahlen, entgegengewirkt.

Optional kann mit einem in den Massedrucksensor 6B integrierten Massetemperatursensor 6C die Temperatur der Kunststoffschmelze in der Viskositätsmessvorrichtung erfasst und daraus eine Temperaturdrift der Schmelzeviskosität errechnet und mittels Software kompensiert werden.

Die zweite, austragseitige Zahnradpumpe 3B erzeugt den nötigen Druck, um die bereits gemessene Schmelze durch die Austragsleitung 16 und die daran anschließende Rückführleitung 17 wieder zurück in den Schmelzekanal des Extruders zu drücken. Es findet somit im Regelfall bei den meisten Anwendungen der Erfindung eine verlustfreie Messung der Viskosität der Kunststoffschmelze statt.

Zu große Zuführrohre 2 bringen den Nachteil mit sich, dass die Verweilzeit des Kunststoffs in der Viskositätsmessvorrichtung vor der Messdüse 5 größer wird und somit eine Viskositätsänderung des Kunststoffs nur zeitversetzt wahrgenommen wird. In der Praxis haben sich Verweilzeiten des Kunststoffes zwischen 30 Sekunden und 5 min bewährt.

Für den Anfahr- und Abstellprozess der erfindungsgemäßen Viskositätsmessvorrichtung weist diese zwei Spülvorrichtungen/Absperrvorrichtungen 9A, 9B auf.

Bei Extrudern und anderen Kunststoffschmelze erzeugenden Einrichtungen, wie z.B. Reaktoren, kann es beim Starten des Prozesses nach längeren Stillständen zur Bildung von Verkokungen aufgrund langer Verweilzeiten des Kunststoffes unter Temperatur- und/oder Sauerstoffeinfluss oder zu sonstigen Verunreinigungen durch Fremdmaterialien kommen.

Gegenüber der Größe der Verkokungen (z.B. Polyester und Polyamide) und der relativ dazu kleinen Zahnradgeometrie der Zahnradpumpen 3A, 3B und des Querschnitts der Messdüse 5 kann es zu Verstopfungen und sogar zum Steckenbleiben der Zahnräder der Zahnradpumpen 3A, 3B kommen.

Aus diesem und weiteren Gründen, wie z.B. das Starten der Viskositätsmessvorrichtung im laufenden Extrusionsprozess und damit dem Pumpen von degradiertem Material aus der Viskositätsmessvorrichtung zurück in den Produktionsprozess, sind zum Starten und Stoppen der Viskositätsmessvorrichtung einlauf- und austragseitige Spül-/Absperrvorrichtungen 9A, 9B vorgesehen, die im einfachen Fall, wie dargestellt, manuell betätigt werden. Es ist jedoch auch eine automatisierte Betätigung mittels Pneumatik, Elektromotoren oder Hydraulik denkbar. Weiter ist optional ein Umschaltventil zwischen einer Wiederverwendung der gemessenen Schmelze im Extrusionsprozess oder der Ableitung der Schmelze möglich. In den Zeichnungen ist die besonders bevorzugte Ausführung eines kombinierten Ableit-/Abspermentils, gebildet aus der entsprechend konfigurierten Verschlussschraube 7 und der Spül-/Absperrvorrichtung 9B, dargestellt.

Um eine Beschädigung der Zahnräder der Zahnradpumpen 3A, 3B im Anfahrprozess auszuschließen, werden zuerst die beiden als Ablassschrauben ausgebildeten Spül-/ Absperrvorrichtungen 9A, 9B geöffnet. Durch den vorhandenen Werkzeugdruck des formgebenden Werkzeugs wird Kunststoffschmelze vom Extruder 30 durch das Zuführrohr 2, die im Gehäuse ausgebildeten Einlaufleitungen 18, die Rückführleitung 17 und die Austragsleitung 16 durch die geöffneten Spül-/Absperrvorrichtungen 9A, 9B hindurch gespült. Dadurch werden die Schmelzeleitungen im Gehäuse 1 gereinigt.

Sobald die Schmelze in der gewünschten Reinheit aus dem Gehäuse 1 austritt, wird die einlaufseitige Spül-/Absperrvorrichtung 9A geschlossen, wodurch Kunststoffschmelze zur einlaufseitigen Zahnradpumpe 3A gedrückt wird. Die austragseitige Spül-/ Absperrvorrichtung 9B ist so eingestellt, dass die Rückführleitung 17 verschlossen ist. Nun werden die beiden Zahnradpumpen 3A, 3B gestartet und die Schmelze durch die Messdüse 5 hindurch und anschließend durch die austragseitige Spül-/Absperrvorrichtung 9B hindurch aus der Viskositätsmessvorrichtung hinaus gefördert.

Erst wenn auch in diesem Betriebszustand die Schmelze in einer einwandfreien Qualität austritt, kann die austragseitige Spül-/Absperrvorrichtung 9B auf Normalbetrieb umgestellt werden und die Schmelze nach erfolgter Viskositätsmessung durch die Rückführleitung 17 hindurch verlustfrei wieder dem Produktionsprozess zugeführt werden.

Die Beheizung des Oberteils 1A, des Mittelteils 1B und des Unterteils 1C des Gehäuses 1 erfolgt über die elektrische Heizeinrichtung 8, welche das Gehäuse 1 umschließt und mittels eines Temperaturreglers auf Betriebstemperatur gehalten wird, auf die sich auch das Gehäuse 1 erwärmt. Der Temperaturregler kann in eine Computersteuerung 23 der Viskositätsmessvorrichtung integriert oder als separate Baugruppe ausgeführt sein. Die Betriebstemperatur wird normalerweise so gewählt, dass die Gehäusetemperatur ungefähr die Verarbeitungstemperatur der Kunststoffschmelze einnimmt, die beispielsweise bei Polyolefinen zwischen 160 und 250°C, bei Polyamiden und Polyestern zwischen 240 und 300°C liegt. Die Betriebstemperatur der Heizeinrichtung 8 wird in jedem Fall jedoch so hoch gewählt, dass sich eine Gehäusetemperatur einstellt, die über der Schmelztemperatur des Kunststoffes liegt, um ein Einfrieren des Kunststoffs im Gehäuse 1 zu verhindern.

Die beiden in Serie geschalteten Zahnradpumpen 3A, 3B werden von dem Antriebsmotor 20, optional unter Zwischenschaltung eines Getriebes 21 angetrieben. Aufgrund der hohen Drehzahlkonstanz und der Rückführung der Drehzahlsignale an eine Regelungseinrichtung bieten sich dafür handelsübliche Servomotoren mit Regler an. Es ist jedoch auch ein frequenzgesteuerter Wechselstrommotor oder Gleichstrommotor mit oder ohne Rückführung von Drehzahlsignalen verwendbar. Der Motor 20 ist über eine Kupplung 22 mit der Antriebswelle 4 lösbar verbunden, wobei die Kupplung 22 auch die temperaturabhängige Längenausdehnung der Welle 4 aufnimmt. Denkbar ist auch eine fixe Zentrierung des Antriebsmotors 20 auf der Antriebswelle 4 mit einer Längendehnung der Antriebswelle 4 in die vom Antrieb abgewandte Seite.

Die Antriebswelle 4 ist über die beiden Gleitlager 10 aus geeigneten handelsüblichen Materialpaarungen zwischen Antriebswelle und Lagermaterial gelagert. Die Toleranz zwischen dem Außendurchmesser der Antriebswelle 4 und dem Innendurchmesser der Lager 10 ist so gewählt, dass es einerseits trotz durch den Schmelzestrom verursachter Temperaturunterschiede zwischen der Antriebswelle 4 und dem Gehäuse 1 zu keinem Festfressen der Antriebswelle 4 in den Lagern 10 kommt, und andererseits das Lagerspiel möglichst klein gehalten wird, um eine verlässliche Abdichtung gegen das Eindringen von Kunststoffschmelze zu bieten.

Auf der Welle 4 sind die einlaufseitige und die austragseitige Zahnradpumpe 3A, 3B mit einer lösbaren Verbindung 11 angebracht, siehe Fig. 4. Als einfachste und besonders bevorzugte Möglichkeit dafür bietet sich ein Passstift 11A an, der den Vorteil einer besonders einfachen Montage und Demontage bringt.

Wie bereits oben erwähnt, weist das Zahnradpaar der austragseitigen Zahnradpumpe 3B einen gegenüber dem Zahnradpaar der einlaufseitigen Zahnradpumpe 3A erhöhten Volumenstrom pro Zeiteinheit auf. Aufgrund der gleichen Drehzahl der Zahnradpaare der Zahnradpumpen 3A, 3B wird der unterschiedliche Volumenstrom pro Zeiteinheit in der bevorzugten Ausführung durch Dickenunterschiede der Zahnräder erreicht, was große Vorteile in der Serienfertigung der Zahnräder mit sich bringt. Es ist jedoch auch denkbar, den Durchmesser beziehungsweise die Zahngeometrie der Zahnräder unterschiedlich auszubilden.

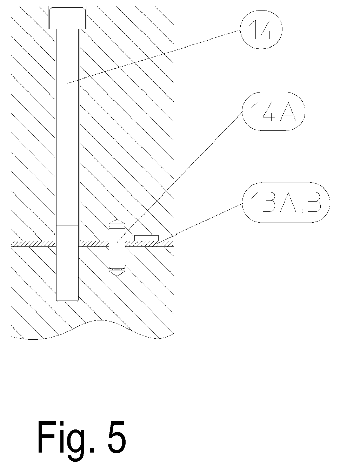

In der besonders bevorzugten Ausführungsform weist das Gehäuse 1 ein Gehäuseoberteil 1A, Gehäusemittelteil 1B und ein Gehäuseunterteil 1C auf. Dabei werden die Trennebenen 12A, 12B der drei Gehäuseteile so gewählt, dass diese im Bereich, vorzugsweise in der Schnittachse, der Zahnräder der Zahnradpumpen 3A, 3B liegen, was eine besonders einfache Fertigung mit sich bringt. Fertigungstechnisch kann es zweckmäßig sein, Scheiben 13A, 13B in der Dicke der Zahnräder vorzusehen und diese beim Zusammenbau des Gehäuses zwischen Oberteil 1A und Mittelteil 1B bzw. Mittelteil 1B und Unterteil 1C mittels Schrauben 14 zu klemmen und mit Passstiften 14A zu positionieren, wie in Fig. 5 gezeigt.

In der besonders bevorzugten Ausführungsform weist das Gehäuse 1 eine zylindrische Gestalt auf, um die kostengünstige Beheizung mittels einer als Rundheizband ausgebildeten Heizeinrichtung 8 zu gewährleisten. Es sind jedoch auch andere Gehäuseformen bedingt durch von der Produktionsmaschine vorgegebenen Platzverhältnissen möglich.

Im Gehäusemittelteil 1B ist die Messdüse 5, in der besonders bevorzugten Ausführungsform eine von außen zugängliche Messdüse 5 in variabler Geometrie, eingeschraubt. Es ist alternativ auch vorgesehen, die Messdüse 5 zu klemmen oder in einer fixen Geometrie direkt in das Grundmaterial des Mittelteils 1B zu integrieren.

Der einlauf- und austragsseitige Schmelzeanschluss der Viskositätsmessvorrichtung an den Extruder ist in einer besonders bevorzugten Ausführungsform der Erfindung so realisiert, indem das Zuführrohr 2 als Doppelzylinder ausgebildet ist, wobei in einem Zylinder die Zuführung der Schmelze aus dem Extruder 30 oder dergleichen in die Viskositätsmessvorrichtung erfolgt und im anderen, koaxial zum ersten Zylinder verlaufenden Zylinder die Rückführung der Schmelze in den Produktionsprozess stattfindet. Diese ineinander laufenden Schmelzeleitungen bringen den erheblichen Vorteil, dass in dem Schmelzeanschluss des Schmelze erzeugenden Bauteils (Extruder 30, Reaktor, etc) lediglich eine Bohrung vorgesehen werden muss. Es sind jedoch auch Ausführungsformen mit getrennten Schmelzezuführ- und rückführleitungen denkbar. Dabei ist jedoch im Speziellen darauf Augenmerk zu legen, dass der Einlauf der Schmelze immer in Schmelzeflussrichtung und der Rücklauf immer stromabwärts davon erfolgt, um zu verhindern, dass die Schmelze mehrfach gemessen wird.

In der besonders bevorzugten dargestellten Ausführung der Erfindung wird die Schmelze mittels einer für die jeweiligen Temperaturen geeigneten Stopfbuchse 15 am Austritt an der Antriebsseite des Gehäuses 1 gehindert.

In der WO 97/10492 ist eine Berechnungsmethode für MVR/MFI in g/min in Abhängigkeit des Volumendurchsatzes und der Düsengeometrie vorgestellt. Diese Berechnungsmethode ist in der vorliegenden Erfindung anwendbar, berücksichtigt jedoch Abweichungen z.B. aufgrund der Rauheit der Messdüse 5 nicht.

Eine besonders pragmatische und praxisorientierte Auswertung bzw. Umrechnung gemäß einer bevorzugten Ausführung der Erfindung ordnet den sich bei einer fixen Drehzahl einstellenden Differenzdruck zwischen Eingang und Ausgang der Messdüse 5, erfasst durch die Massedrucksensoren 6A, 6B, mittels einer Tabelle 25 den für den jeweiligen Kunststoff üblichen Viskositätseinheiten zu, die mittels Software in die Tabelle eingegeben und ausgelesen werden.

Zu diesem Zweck ist eine Computersteuerung 23 vorgesehen, die einen Prozessor und einen Programmspeicher aufweist, um die Software zur Steuerung der Viskositätsmessvorrichtung abzuarbeiten. Die Computersteuerung 23 ist mit einem Datenspeicher 24 verbunden, in dem die Tabelle 25 gespeichert wird. Weiters ist die Computersteuerung 23 mit den Massedrucksensoren 6A, 6B, den Temperatursensoren 6C, 8A, der Heizeinrichtung 8 und dem Motor 20 verbunden.

### Beispiel:

In einem PET Extruder werden fünf verschiedene PET-Materialien mit einer Ausgangsviskosität (vor der Extrusion) von 0,6 dl/g, 0,65 dl/g, 0,7 dl/g, 0,75 und 0,8 dl/g nacheinander verarbeitet. Nach einer gewissen Verzögerungszeit stellt sich auf der Viskositätsmessvorrichtung bei konstanter Drehzahl jeweils ein konstanter Differenzdruck (Delta P), gemessen mittels der Massedrucksensoren 6A, 6B ein. Dieser Wert wird notiert und zum selben Zeitpunkt ein Muster des Halbfertig- oder Fertigproduktes nach der Extrusionsanlage gezogen, die einer genormten Labormessung zugeführt wird. Diese Werte der intrinsischen Viskosität (iV) werden anschließend in der Tabelle 25 mittels der Computersteuerung 23 der Viskositätsmessvorrichtung eingegeben.

**Tabelleneingabefeld an der Computersteuerung 23 der Viskositätsmessvorrichtung:**

| Material Start iV | Delta P Messmaschine | Produkt-iV gemäß Labormessung |
|---|---|---|
| 0,6 | 3 | 0,57 |
| 0,65 | 6 | 0,61 |
| 0,7 | 12 | 0,66 |
| 0,75 | 20 | 0,70 |
| 0,8 | 30 | 0,74 |

Die in der Computersteuerung 23 laufende Software der Viskositätsmessvorrichtung interpoliert nach Eingabe der Tabelle und berechnet die angezeigte intrinsische Viskosität aus dem Differenzdruck delta p und den inteipolierten Werten der aufgezeichneten und programmierten Tabelle 25, was in der Regel zu einer ausreichend genauen Anzeige führt. Es ist auch denkbar, die gleichen Messungen mit unterschiedlichen Massetemperaturen (ermittelt durch den Massetemperatursensor 6C) zu wiederholen und die Schmelzetemperatur als Kompensationswert zusätzlich anzuwenden. Es kann auch zweckmäßig sein, bei abweichenden Schmelzetemperaturen die Drehzahl der Viskositätsmessvorrichtung aufgrund von Erfahrungswerten zu erhöhen oder zu erniedrigen.

Eine gänzlich andere, ebenfalls in der Erfindung anwendbare Methode ist, die Viskositätsmessvorrichtung im Konstantdruckverfahren bei vorgewähltem Druck delta p zu betreiben und über die sich einstellende Drehzahl der Welle 4 auf die Viskosität mit dem oben beschriebenen Verfahren rückzuschließen.

## Patentansprüche

1. Vorrichtung zur Messung der Viskosität von Flüssigkeiten, insbesondere von thermoplastischen Kunststoffschmelzen, mit einem Gehäuse (1), in dem zumindest eine einlaufseitige Zahnradpumpe (3A) zur Förderung der Flüssigkeit angeordnet ist, wobei das Gehäuse (1) ein Zuführrohr (2) aufweist, durch das der einlaufseitigen Zahnradpumpe (3A) die Flüssigkeit zuführbar ist, mit einem Antriebsmotor (20), der eine Welle (4) antreibt, welche Welle (4) zumindest ein Zahnrad der Zahnradpumpe (3A) antreibt, mit einem im Flüssigkeitspfad stromabwärts von der einlaufseitigen Zahnradpumpe (3A) angeordneten Messdüse (5), vor deren Eingang ein einlaufseitiger Massedrucksensor (6A) angeordnet ist, **dadurch gekennzeichnet, dass** das Gehäuse (1) einen Oberteil (1A), in den das Zuführrohr (2) mündet, einen Mittelteil (1B) und einen Unterteil (1C) aufweist, die als separate Gehäuseteile ausgebildet sind, wobei der Oberteil (1A) mit dem Mittelteil (1B) und der Mittelteil (1B) mit dem Unterteil (1C) mit Verbindungselementen lösbar verbindbar sind, wobei die einlaufseitige Zahnradpumpe (3A) entweder im Oberteil (1A) oder im Mittelteil (1B) angeordnet ist und die Messdüse (5) im Mittelteil (1B) angeordnet ist, wobei der Oberteil (1A), der Mittelteil (1B) und der Unterteil (1C) des Gehäuses (1) so konfiguriert sind, dass im getrennten Zustand die einlaufseitige Zahnradpumpe (3A) und die Messdüse (5) von außen zugänglich sind.

2. Vorrichtung nach Anspruch 1, **dadurch gekennzeichnet, dass** entweder im Mittelteil (1B) oder im Unterteil (1C) des Gehäuses (1) eine von der Welle (4) angetriebene austragsseitige Zahnradpumpe (3B) angeordnet ist, wobei die angetriebene austragsseitige Zahnradpumpe (3B) im Flüssigkeitspfad stromabwärts von der Messdüse (5) angeordnet ist und wobei die austragseitige Zahnradpumpe (3B) von außen zugänglich ist, wenn der Mittelteil (1B) vom Unterteil (1C) des Gehäuses (1) getrennt ist.

3. Vorrichtung nach Anspruch 1 oder 2, **dadurch gekennzeichnet, dass** nach dem Ausgang der Messdüse (5) ein austragseitiger Massedrucksensor (6B) angeordnet ist, wobei eine Computersteuerung (23) dazu ausgebildet ist, einen Differenzdruck (delta P) aus den Drucksignalen des einlaufseitigen Massedrucksensors (6A) und des austragseitigen Massedrucksensors (6B) zu ermitteln.

4. Vorrichtung nach Anspruch 3, **dadurch gekennzeichnet, dass** sie einen Massetemperatursensor (6C) aufweist, der vorzugsweise in den austragseitigen Massedrucksensor (6B) integriert ist.

5. Vorrichtung nach einem der vorhergehenden Ansprüche, **gekennzeichnet durch** eine Heizvorrichtung (8) zur Beheizung des Gehäuses (1), wobei die Heizvorrichtung (8) vorzugsweise als an der Gehäuseaußenfläche angeordnetes elektrisches Heizband ausgebildet ist.

6. Vorrichtung nach Anspruch 5, **gekennzeichnet durch** einen Temperatursensor (8A), der die Temperatur des Gehäuses (1) erfasst und dessen Ausgangsignal an einen Regler, vorzugsweise die Computersteuerung (23) zur Regelung der Heizvorrichtung (8) rückgekoppelt ist.

7. Vorrichtung nach einem der vorhergehenden Ansprüche, **dadurch gekennzeichnet, dass** der Oberteil (1A), der Mittelteil (1B) und der Unterteil (1C) des Gehäuses (1) in Ebenen (12A, 12B) voneinander trennbar sind, die im Bereich der Zahnräder der Zahnradpumpen (3A, 3B) liegen, wobei vorzugsweise Scheiben (13A, 13B) zwischen dem Oberteil (1A) und dem Mittelteil (1B) bzw. zwischen dem Mittelteil (1B) und dem Unterteil (1C) angeordnet sind.

8. Vorrichtung nach einem der Ansprüche 2 bis 7, **dadurch gekennzeichnet, dass** die einlaufseitige Zahnradpumpe (3A) und die austragseitige Zahnradpumpe (3B) so konfiguriert sind, dass die austragsseitige Zahnradpumpe (3B) einen größeren Volumenstrom pro Zeiteinheit aufweist als die einlaufseitige Zahnradpumpe (3A).

9. Vorrichtung nach Anspruch 8, **dadurch gekennzeichnet, dass** die Zahnräder der austragseitigen Zahnradpumpe (3B) dicker oder mit einem größeren Durchmesser ausgebildet sind als die Zahnräder der einlaufseitigen Zahnradpumpe (3A).

10. Vorrichtung nach einem der vorhergehenden Ansprüche, **dadurch gekennzeichnet, dass** die Messdüse (5) austauschbar im Gehäuse (1) angeordnet ist.

11. Vorrichtung nach einem der vorhergehenden Ansprüche, **gekennzeichnet durch** eine Rückführleitung (17), **durch** die Flüssigkeit nach der Messung verlustfrei in einen Produktionsprozess rückführbar ist.

12. Vorrichtung nach Anspruch 11, **dadurch gekennzeichnet, dass** das Zuführrohr (2) als Doppelzylinder ausgebildet ist, wobei in einem Zylinder die Zuführung der zu messenden Flüssigkeit in das Gehäuse (1) und im anderen, koaxial zum ersten Zylinder verlaufenden zweiten Zylinder die Rückführung der gemessenen Flüssigkeit in den Produktionsprozess stattfindet.

13. Vorrichtung nach einem der Ansprüche 3 bis 12, **dadurch gekennzeichnet, dass** die Computersteuerung (23) dazu konfiguriert ist, den von ihr ermittelten Differenzdruck (delta P) in Viskositätswerte umzurechnen, insbesondere mittels Interpolation.

14. Vorrichtung nach Anspruch 13, **dadurch gekennzeichnet, dass** die Computersteuerung (23) Zugriff auf eine in einem Speicher (24) gespeicherte Tabelle (25) hat, in der Umrechnungswerte zwischen dem Differenzdruck (delta P), den von der Vorrichtung ermittelten intrinsischen Viskositätswerten und mittels Labormessungen ermittelter Vergleichwerte der intrinsischen Viskosität gespeichert sind, wobei die Computersteuerung dazu programmiert ist, aus dem gemessenen Differenzdruck (delta P) und den zugehörigen intrinsischen Viskositätswerten in der Tabelle einen resultierenden Wert der intrinsischen Viskosität der Flüssigkeit zu interpolieren.
